# EUROPÄISCHE PATENTSCHRIFT

(11) **EP 1 666 015 B1**
(45) Veröffentlichungstag und Bekanntmachung des Hinweises auf die Patenterteilung: **02.01.2008**
(21) Anmeldenummer: 05022835.2
(22) Anmeldetag: 20.10.2005
(51) Int. Cl.: A61G 9/02, A61L 2/26

(54) **Gerät zur Reinigung und Desinfektion von Stechbecken und Urinflaschen**
Apparatus for cleaning and disinfecting bed pans and urinals
Dispositif pour nettoyage et desinfektion des bassins hygiéniques et urinals

(30) Priorität: 11.11.2004 DE 102004054413
(43) Veröffentlichungstag der Anmeldung: 07.06.2006
(73) Patentinhaber: BG Edelstahl und Kunststofftechnik für Krankenhaus Industrie und Wasserwirtschaft GmbH, 45657 Recklinghausen (DE)
(72) Erfinder: Barselak, Peter, 44581 Castrop-Rauxel (DE)
(74) Vertreter: Gesthuysen, von Rohr & Eggert

(56) Entgegenhaltungen:
- EP-A- 0 093 846
- EP-A- 1 354 577
- DE-A1- 19 509 877
- GB-A- 401 375

## Beschreibung

Die Erfindung betrifft ein Gerät zur Reinigung und Desinfektion mit einem Gerätegehäuse, bestehend aus einer Rückwand, zwei Seitenwänden, einer Boden- und einer Deckenwand sowie mit einer verschließbaren Frontklappe, mit im Innenraum angeordneten Sprühdüsen zum Reinigen von Urinflaschen und mit einer sich nach dem Prinzip eines Segnerschen Wasserrades durch Rückstoßimpulse drehenden Sprühvorrichtung zur Reinigung eines Stechbeckens sowie mit einem Dampfeinlaß zur Desinfektion.

Derartige Geräte sind seit vielen Jahren bekannt, z. B. aus EP-A-0 093 846 und auch aus dem Prospekt der Anmelderin mit dem Titel "BEW-Stechbecken-Reinigungs- und Desinfektionsautomaten Pflegearbeitsraum - Ausstattung". Im Innenraum derartiger Geräte können mehrere Urinflaschen und ein Stechbecken oder der Topf eines Stuhlklosetts an Halterungen der Frontklappe gehaltert werden und nach deren Schließen von den Sprühdüsen sowie der Sprühvorrichtung besprüht werden. Dabei ist die Bodenwand des Gerätegehäuses trichterförmig ausgebildet, um die herausgespülten Fäkalien rasch nach unten in einen Abwasserkanal leiten zu können.

Die Anschlüsse für die Sprühdüsen und die Sprühvorrichtung sind an der Außenseite der Rückwand des Gerätegehäuses angeordnet und bilden ein spinnenartiges Rohrsystem, welches mit der jeweiligen Wasser- sowie mit der Dampfzufuhr verbunden ist. Abgesehen von dem nicht unbeträchtlichen Verrohrungsaufwand an der Außenseite der Rückwand des Gerätegehäuses birgt eine solche Verrohrung die Gefahr einer Undichtigkeit an einer der Verschraubungen bzw. einer der Löt- oder Quetschverbindungen mit der Folge, daß dann das gesamte Gerät stillgesetzt und erst nach einer entsprechenden Reparatur zur Vermeidung von weitergehenden Wasserschäden in Betrieb genommen werden kann. Ein weiteres Problem bildet die Reinigung eines Stechbeckens und eines Topfes an seiner Außenwandung, da diese von den Strahlen der sich drehenden Sprühvorrichtung aufgrund des vorstehenden Kragens oder Randes an der Oberseite nicht oder nur äußerst schlecht erreicht werden kann. Damit bleibt eine vollständige Reinigung und daher auch eine vollständige Desinfektion dieser Teilbereiche dem Zufall überlassen.

Hier setzt nun die Erfindung ein. Dieser liegt die Aufgabe zugrunde, ein Gerät der eingangs genannten Gattung zu schaffen, welches bei minimaler Verrohrung an der Außenseite der Rückwand des Gerätegehäuses eine ebenso gleichmäßige wie effektive Reinigung durch die Sprühdüsen und die Sprühvorrichtung sowie auch eine wirksame Desinfektion durch Dampf bei minimalem Bau- und Montageaufwand gewährleistet.

Diese Aufgabe wird in Verbindung mit dem eingangs genannten Gattungsbegriff erfindungsgemäß dadurch gelöst, daß im Innenraum des Gerätegehäuses ein kompaktes Verteilergehäuse angeordnet ist, welches als Druckkammer ausgebildet ist und an mindestens einer Außenfläche mit mehreren Sprühdüsen und mindestens einer Sprühvorrichtung versehen ist sowie an seiner Rückseite zwei Anschlußstutzen für Wasser und Dampf aufweist, die abgedichtet durch die Rückwand des Gerätegehäuses nach außen hindurchgeführt und auf der Außenseite dieser Rückwand mit nur einer Wasser- und einer Dampfzufuhr strömungsleitend verbunden sind. Aufgrund eines nunmehr im Innenraum des Gerätegehäuses angeordneten als Druckkammer ausgebildeten Verteilergehäuses sind an der Rückwand des Gerätegehäuses nur noch zwei Anschlüsse, nämlich einer für Wasser und einer für Dampf erforderlich. Der Anschluß für Wasser ist derart ausgebildet, daß nach Beaufschlagung des Innenraumes der Druckkammer mit Reinigungswasser in dieser ein konstanter Druck aufbaut wird, wodurch sämtliche von dieser Druckkammer abgehenden Düsen und Sprühvorrichtungen mit einem gleichmäßigen Druck beaufschlagt werden. Gesonderte Rohrleitungen zu den einzelnen Düsen bzw. der Sprühvorrichtung entfallen. Es sind daher nur noch zwei Zufuhrleitungen auf der Rückwand des Gerätegehäuses abzudichten, nämlich einmal der Anschlußstutzen für die Wasserzufuhr und ein weiterer Anschlußstutzen für die Dampfzufuhr. Sollte wider Erwarten einer dieser Anschlüsse undicht werden, kann dies sofort festgestellt und unverzüglich behoben werden, ohne daß es, wie bislang bei der an der Rückwand angeordneten "Rohrspinne", zur Untersuchung auf Dichtigkeit einer jeden Rohrverbindung ankommt. Aufgrund der gleichmäßigen Druckbeaufschlagung des Innenraumes der Druckkammer mit Wasser ist auch eine gleichmäßige Reinigung der Urinflaschen und der Stechbecken gewährleistet.

Nach einer vorteilhaften Weiterbildung der Erfindung ist der Anschlußstutzen für die Dampfzufuhr druckdicht mit einem Rohr verbunden, welches dampf- und wasserdicht durch die Druckkammer hindurchgeführt ist und an einer abgedichteten Öffnung an deren Frontseite endet. Dadurch wird der Dampf durch die Druckkammer hindurchgeführt, ohne daß er in diese eindringen kann oder umgekehrt dieses Dampfrohr von Wasser beaufschlagt werden kann.

Um ein Eindringen von Fäkalien in dieses Dampfrohr während der Sprühphase mit Wasser zu unterbinden, ist dieses Rohr vom Anschlußstutzen zur Frontseite der Druckkammer in Richtung auf die Bodenwand des Gerätegehäuses geneigt. Dadurch kann eindringendes Wasser jederzeit unter seiner Schwerkraft aus der Öffnung an der Frontseite herausfließen.

Vorteilhaft ist der Anschlußstutzen für die Wasserzufuhr abgedichtet in den Innenraum der Druckkammer geleitet, in welcher bei Beaufschlagung mit dem Reinigungswasser ein gleichmäßiger Druck über deren gesamten Innenraum aufgebaut ist. Dadurch wird eine ungleichmäßige Druckbeaufschlagung der einzelnen Sprühdüsen oder gar der Sprühvorrichtung durch unterschiedliche Druckverluste - wie beim Stand der Technik - innerhalb eines zuführenden Rohrleitungsnetzes unterbunden.

Das prismatische Verteilergehäuse weist im Querschnitt eine Dreieckform auf, dessen Basisseite mit den Anschlußstutzen für Wasser und Dampf versehen ist sowie auf der Innenseite der Gehäuserückwand befestigt ist. Die Befestigung kann beispielsweise dadurch erfolgen, daß die Anschlußstutzen für die Dampf und Wasserzufuhr auf der Außenseite der Gehäuserückwand durch entsprechende Schraubmuffen gegen die Rückwand des Verteilergehäuses verspannt werden mit der Folge, daß die Rückwand des Verteilergehäuses gegen die Innenseite dieser Gehäuserückwand gezogen wird.

Nach einer vorteilhaften Weiterbildung der Erfindung weist die den Urinflaschen und dem Stechbecken zugewandte Querschnitt-Dreieckseite des Verteilergehäuses eine Trapezform auf, dessen längerer Basisseite der Rückwand des Gerätegehäuses zugekehrt ist.

Die Frontseite des Verteilergehäuses ist rechteckig ausgebildet, wohingegen die beiden Seitenflächen des Verteilergehäuses dreieckförmig ausgebildet sind und die kürzeren Schenkelseiten dieser Dreiecke zugleich die Schmalseiten der rechteckigen Frontseite bilden. Dadurch weist das Verteilergehäuse und damit die Druckkammer die Form eines Prismas auf, dessen dreieckförmige Grundfläche und dessen dreieckförmige Dachfläche nicht zueinander parallel verlaufen, sondern von der schmalen Dreieckseite an der Frontplatte ausgehend zueinander divergieren.

Nach einer besonders vorteilhaften Weiterbildung der Erfindung ist die Sprühvorrichtung auf der trapezeodalen Oberseite des Verteilergehäuses als Sprühkopf ausgebildet und weist eine Birnenform auf, die mit mindestens vier Sprühschlitzen versehen ist, von denen zwei parallel, jedoch exzentrisch zur Drehachse des Sprühkopfes verlaufen. Durch diese Ausbildung als birnenförmiger Sprühkopf - und nicht mehr als zweiarmiges Segnersches Wasserrad, wie beim Stand der Technik - steigt die Drehzahl der Sprühvorrichtung infolge der Rückstoßimpulse gegenüber den bisher bekannten Sprühvorrichtungen erheblich an, mit der Wirkung einer stark vergrößerten Reinigungs- und Sprühwirkung aufgrund geringerer Druckverluste.

Vorteilhaft weist die trapezförmige Oberseite des Verteilergehäuses eine solche Neigung in Richtung auf die an der Frontklappe gehalterten Stechbecken und Urinflaschen auf, daß der Sprühkopf senkrecht zur Bodenfläche des Stechbeckens und die Sprühdüsen senkrecht zu der Bodenfläche der jeweiligen Urinflasche ausgerichtet sind. Dadurch ist eine tiefgreifende Reinigung auch von Bodensätzen in den vorgenannten Behältern gewährleistet.

Weiterhin ist für die Reinigung der Außenflächen der Urinflaschen und des Stechbeckens in dem Verteilergehäuse mindestens eine Leitung mit mindestens einer Sprühdüse und/oder einem Sprühkopf zur Deckenwand des Gerätegehäuses geführt. Dadurch erfolgt auch eine entsprechend effektive Besprühung und Reinigung der Außenflächen der zu reinigenden Behälter. Diese Leitung kann alternativ auch an der Außenseite der Rückwand verlegt werden.

Um speziell die Außenflächen der mit einem Kragen versehenen Stechbecken gründlich reinigen zu können, sind etwa senkrecht auf den dreieckförmigen Seitenflächen des Verteilergehäuses zwei Sprühköpfe mit einer Strahl-Leitvorrichtung für die austretenden Wasserstrahlen angeordnet, die zielgenau auf die beiden gegenüberliegenden Außenseiten des Stechbeckens gerichtet sind. Durch diese zusätzlichen Sprühköpfe an der Deckenwand des Gerätegehäuses sowie an den beiden dreieckförmigen Seitenflächen des Verteilergehäuses erfolgt eine ebenso gründliche wie effektive Reinigung auch der Außenflächen der zu reinigenden Behälter.

Das Verteilergehäuse ist aus Edelstahl hergestellt, wohingegen die in die entsprechenden Öffnungen des Verteilergehäuses abgedichtet, eingeschraubten Sprühdüsen entweder aus Edelstahl oder aus einer verchromten Messinglegierung bestehen.

Ein Ausführungsbeispiel der Erfindung ist in den Zeichnungen dargestellt. Dabei zeigen:
Figur 1 eine perspektivische Ansicht auf das Gerätegehäuse mit eingebautem Verteilergehäuse im Innenraum, ohne Frontklappe,
Figur 2 die perspektivische Ansicht von Figur 1 mit Frontklappe und einem daran gehaltertem Stechbecken,
Figur 3 die Rückansicht des Gerätegehäuses in Richtung des Pfeils III von Figur 2,
Figur 4 die Frontansicht in Richtung des Pfeils IV des Gerätegehäuses von Figur 2 bei geschlossener Frontklappe,
Figur 5 die Draufsicht auf das Gerätegehäuse in Richtung des Pfeils V von Figur 4,
Figur 6 eine Perspektivansicht des prismatischen Verteilergehäuses mit einer daran angeschlossenen zur Deckenwand des Gerätegehäuses geführten Leitung mit mindestens einer Sprühdüse und/oder einem Sprühkopf,
Figur 7 die Seitenansicht des Verteilergehäuses in Richtung des **Pfeils VII von Figur 6,**
Figur 8 die Ansicht in Richtung des Pfeils VIII von Figur 2 auf die Frontseite des Gerätegehäuses mit einem eingesetzten Stechbecken sowie den Sprühdüsen und der Sprühvorrichtung im Reinigungsbetrieb.
Figur 9 die Seitenansicht Richtung des Pfeils IX von Figur 8,
Figur 10 eine Perspektivansicht der Sprühvorrichtung,
Figur 11 die Ansicht der Sprühvorrichtung in Richtung des Pfeils XI von Figur 10,
Figur 12 die Ansicht der Sprühvorrichtung in Richtung des Pfeils XII von Figur 10,
Figur 13 die Draufsicht in Richtung des Pfeils XIII von Figur 11,
Figur 14 eine perspektivische Ansicht eines der beiden Sprühköpfe, die senkrecht auf den dreieckförmigen Seitenflächen des Verteilergehäuses angeordnet sind,
Figur 15 die Ansicht des Sprühkopfes von Figur 14 in Richtung des Pfeils XV,
Figur 16 die Ansicht des Sprühkopfes von Figur 14 in Richtung des Pfeils XVI von Figur 14 und
Figur 17 die Draufsicht auf diesen Sprühkopf in Richtung des Pfeils XVII von Figur 15.

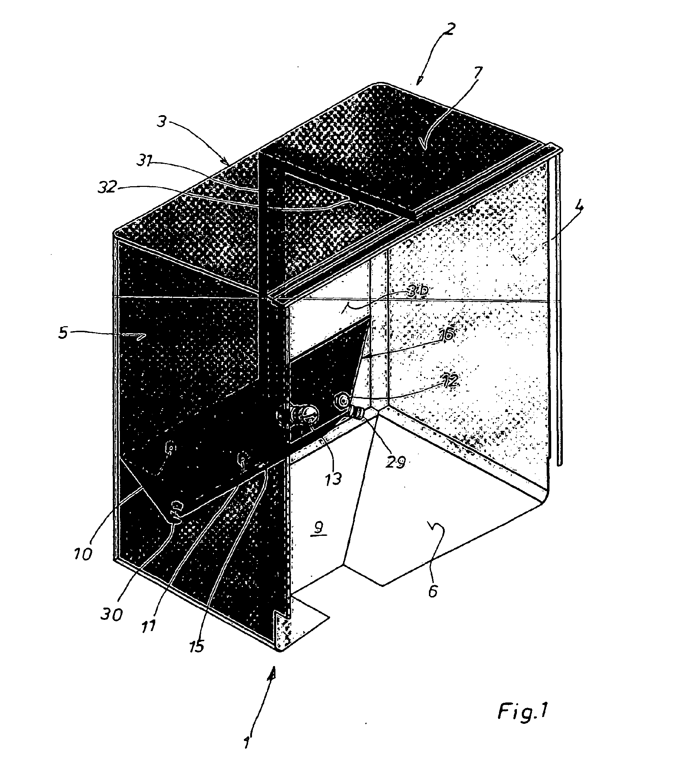

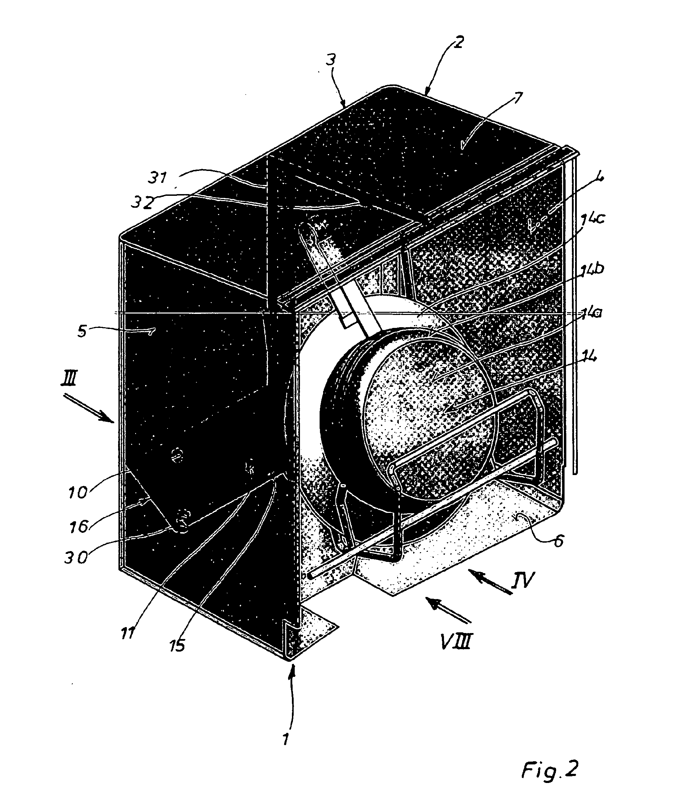

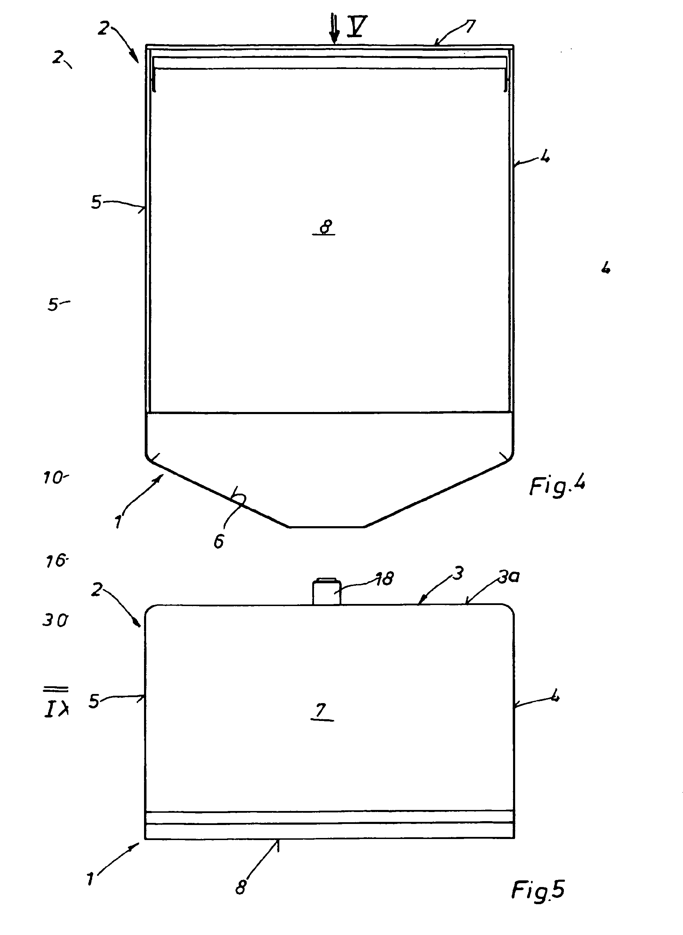

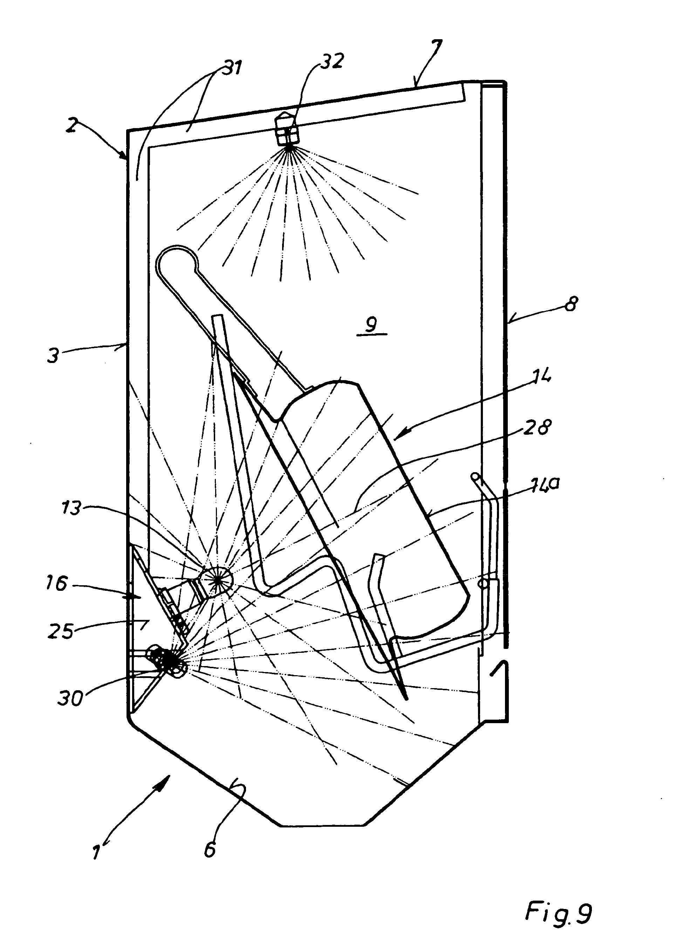

Gemäß den Figuren 1 und 2 setzt sich das Gerät 1 zur Reinigung und Desinfektion aus einem Gehäuse 2 mit einer Rückwand 3 aus zwei Seitenwänden 4, 5, einer trichterförmigen Bodenwand 6, einer Deckenwand 7 sowie einer Frontklappe 8 (siehe Figuren 4 und 9) zusammen. Im Innenraum 9 (siehe Figur 1) sind drei Sprühdüsen 10, 11, 12 zum Reinigen von nicht dargestellten Urinflaschen sowie eine sich nach dem Prinzip eines Segnerschen Wasserrades durch Rückstoßimpulse drehende Sprühvorrichtung 13 zur Reinigung eines Stechbeckens 14 sowie ein Dampfauslaß 15 zur Desinfektion angeordnet.

Die Sprühdüsen 10, 11, 12 und die Sprühvorrichtung 13 sowie der Dampfeinlaß 15 sind im Innenraum 9 des Gerätegehäuses 2 an einem kompakten Verteilergehäuse 16 vorgesehen, welches als Druckkammer ausgebildet ist.

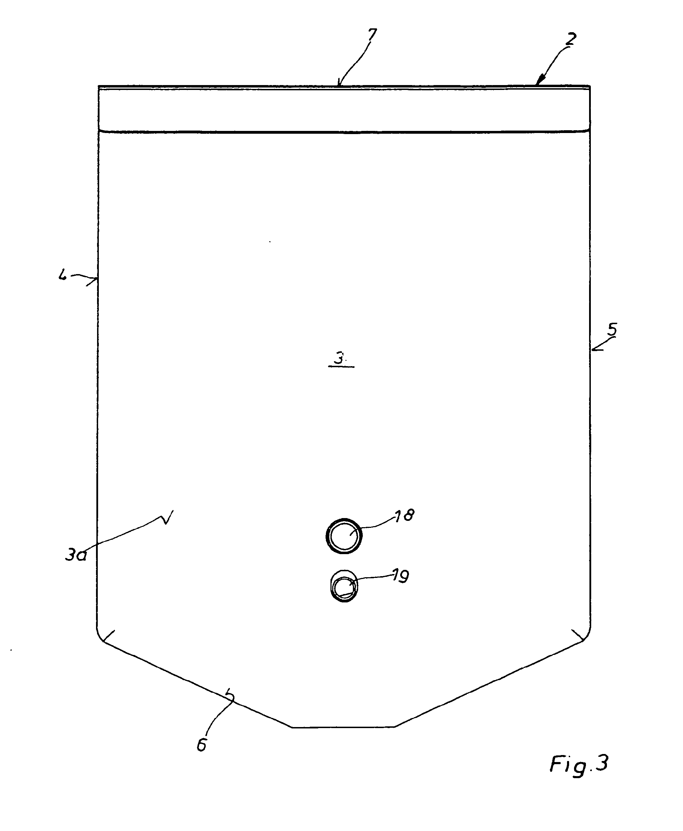

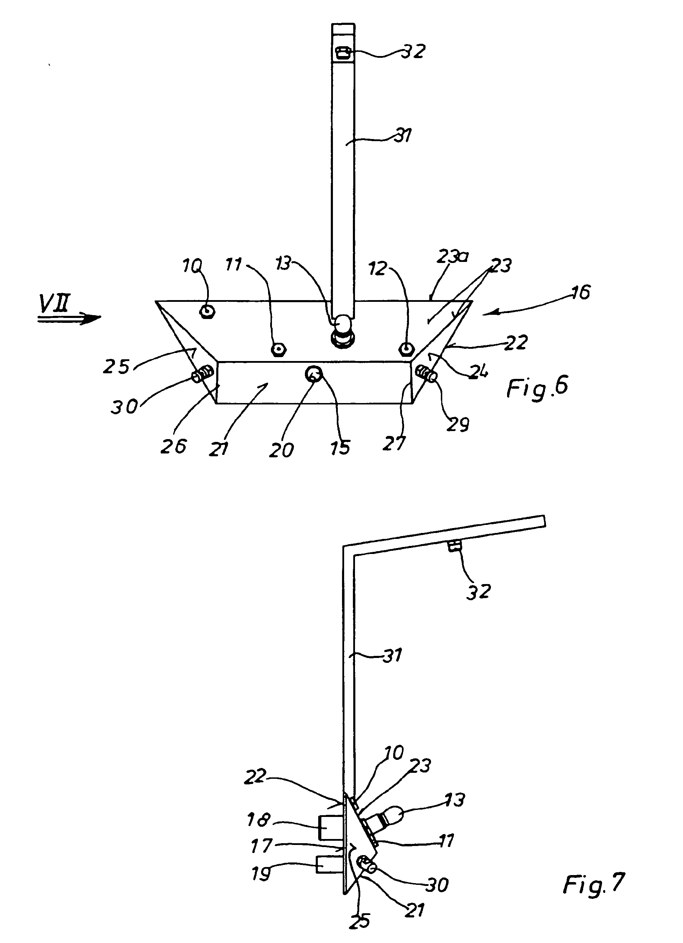

Gemäß den Figuren 3 und 7 sind an der Rückseite 17 des Verteilergehäuses 16 zwei Anschlußstutzen 18, 19 angeordnet, die abgedichtet durch die Rückwand 3 des Gerätegehäuses 2 hindurchgeführt und auf der Außenseite 3a der Rückwand 3 mit einer nicht dargestellten Wasser- und einer Dampfzufuhr strömungsleitend verbunden sind. Die Anschlußstutzen 18, 19 sind mit entsprechenden Außengewinden versehen und werden mit entsprechenden gleichfalls nicht dargestellten Muffen derart verschraubt, daß einerseits diese Anschlussstellen dicht sind und andererseits die Rückseite 17 des Verteilergehäuses 16 fest gegen die Innenseite 3b der Rückwand 3 (siehe Figuren 1 und 7) des Gerätegehäuses 2 gezogen und daran fixiert ist.

Der Anschlußstutzen 19 für die Dampfzufuhr ist druckdicht mit einem Rohr 20 verbunden, welches dampf- und wasserdicht durch die Druckkammer 16 hindurchgeführt ist und an einer abgedichteten Öffnung 20 an deren Frontseite 21 endet (siehe Figur 6). Dieses Rohr 20 ist vom Anschlußstutzen 19 zur Frontseite 21 der Druckkammer 16 in Richtung auf die Bodenwand 6 des Gerätegehäuses 2 geneigt, wodurch eventuell hineingedrückte Verschmutzungen unter ihrer Schwerkraft und/oder infolge des Dampfdurchtrittes wieder in den Innenraum 9 des Gerätegehäuses 2 zurückfließen bzw. zurückgespült werden können.

Der Anschlußstutzen 18 für die Wasserzufuhr ist in den Innenraum der Druckkammer 16 geführt, in welcher bei Beaufschlagung mit Reinigungswasser ein gleichmäßiger Druck über den gesamten Innenraum aufgebaut wird.

Gemäß der Figuren 6 und 7 weist das Verteilergehäuse 16 eine prismatische Form auf und ist im Querschnitt mit einer Dreiecksform versehen, dessen Basisseite 22 mit den Anschlußstutzen 18, 19 für Wasser und Dampf versehen ist, wohingegen die den Urinflaschen und dem Stechbecken 14 zugewandte eine Querschnitts-Dreieckseite 23 des Verteilergehäuses 16 eine Trapezform aufweist, dessen längere Basisseite 23a der Rückwand 3 des Gerätegehäuses 2 zugekehrt ist.

Die Frontseite 21 des Verteilergehäuses 16 ist rechteckförmig ausgebildet. Von den beiden dreieckigen Seitenflächen 24 und 25 des Verteilergehäuses 16 bilden die kürzeren Schenkelseiten 26, 27 zugleich die Schmalseiten 26, 27 der rechteckigen Frontseite 21.

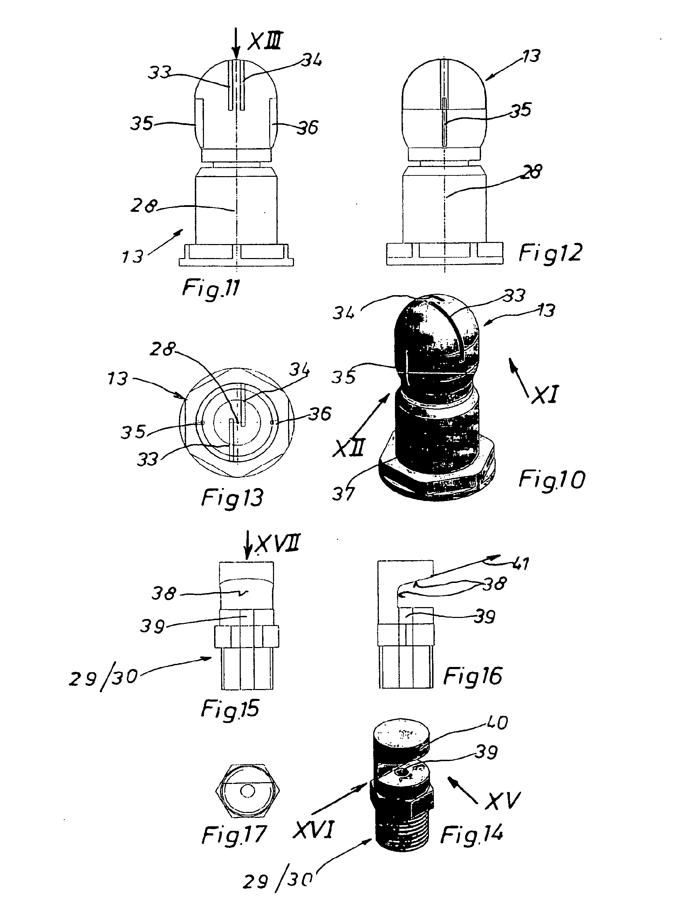

Die Sprühvorrichtung 13 auf der trapezeodalen Oberseite 23 des Verteilergehäuses 16 ist als Sprühkopf ausgebildet und weist eine Birnenform auf, die mit mindestens vier Sprühschlitzen 33 ÷ 36 (siehe Figuren 10 ÷ 12) versehen ist, von denen zwei 33, 34 exzentrisch zur Drehachse 28 des Sprühkopfes 13 verlaufen.

Wie aus den Figuren 6 und 7 in Verbindung mit Figur 2 verdeutlicht wird, weist die trapezförmige Oberfläche 23 des Verteilergehäuses 16 eine solche Neigung in Richtung auf die an der Frontklappe 8 gehalterten Stechbecken 14 und die nicht dargestellten Urinflaschen auf, daß der Sprühkopf 13 mit seiner Drehachse 28 (siehe Figur 9) senkrecht zur Bodenfläche 14a des Stechbeckens und die Sprühdüsen 10 ÷ 12 senkrecht zur Bodenfläche der jeweiligen Urinflasche ausgerichtet sind. Dadurch treffen die Sprühstrahlen mit großer Wucht senkrecht auf die jeweiligen Böden 14a, die sodann als Verteilerplatte wirken, von denen die Sprühstrahlen zu den Innenwänden reflektiert und gemeinsam mit den Schmutzpartikeln in den Innenraum 9 des Gerätegehäuses 2 zurückgedrückt werden.

Jedoch kann von den Sprühdüsen 10 ÷ 12 sowie vom Sprühkopf 13 nicht die Außenseite 14b des Stechbeckens 14 erreicht werden, weil diese Fläche von einem flanschartigen Rand 14c (siehe Figur 2) verdeckt wird. Um gleichwohl auch diese Fläche 14b gründlich in der erforderlichen Weise reinigen zu können, sind senkrecht auf den beiden Dreieckflächen 24, 25 des prismatischen Verteilergehäuses 16 zwei Sprühköpfe 29, 30 mit einer Strahl-Leitvorrichtung 38 für den austretenden Wasserstrahl angeordnet. Diese Strahl-Leitvorrichtung 38 sorgt dafür, daß der Wasserstrahl exakt auf die Außenfläche 14b und die Unterseite des flanschartigen Randes 14c sowie auf die Außenseite des Bodens 14a gerichtet ist, wodurch auch eine gründliche Reinigung dieser bisher problematischen Flächen ermöglicht wird. Dieser Reinigungseffekt wird noch dadurch vervollständigt, daß aus dem Verteilergehäuse 16 mindestens eine Leitung 31 mit mindestens einer Sprühdüse 32 und/oder einem Sprühkopf 13 zur Deckenwand 7 des Gerätegehäuses 2 geführt ist. Auch diese Leitung 31 ist im Innenraum 9 des Gerätegehäuses 2 angeordnet.

Sowohl das Verteilergehäuse 16 als auch die Leitung 31 und das Rohr 20 sind aus Edelstahl hergestellt. Die in die entsprechenden Öffnungen des Verteilergehäuses 16 abgedichtet, eingeschraubten Sprühdüsen 10 ÷ 12, die Sprühdüse 32 in der Leitung 31 und die Sprühvorrichtung 13 sind entweder gleichfalls aus Edelstahl oder aus einer verchromten Messinglegierung.

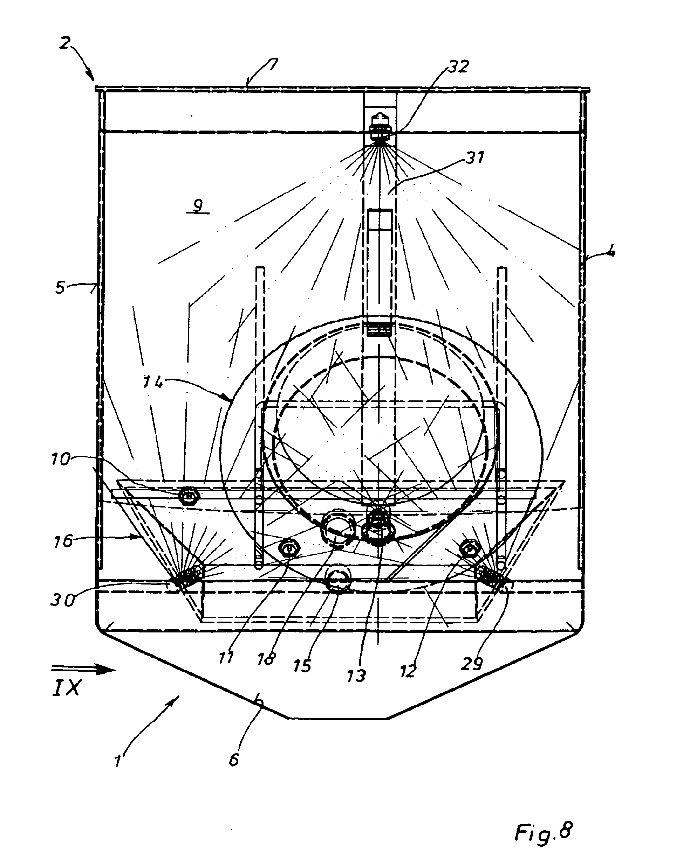

Wie aus den Figuren 8 und 9 entnommen werden kann, sorgen die Sprühdüsen 10 ÷ 12 für die gründliche Reinigung der jeweiligen Innenräume der nicht dargestellten Urinflaschen und die Sprühvorrichtung 13 für die entsprechend gründliche Reinigung der Innenflächen des Stechbeckens 14. Die weiteren Sprühköpfe 29, 30 und 32 sorgen für eine gründliche Reinigung der Außenflächen 14b, 14c des Stechbeckens 14 und der Urinflaschen.

Nach Abschluß des Waschprogramms erfolgt die Desinfektion dadurch, daß durch den Rohrstutzen 19 Dampf unter einem geringen Überdruck in den Innenraum 9 des Gerätegehäuses 2 geleitet wird, wo sich dieser Dampf vollständig sowie gleichmäßig in diesem Innenraum 9 ausbreitet, weil nunmehr der Innenraum 9 als Überdruckkammer für den Dampf dient.

In den Figuren 10 bis 13 ist die Sprühvorrichtung 13 zum Reinigen eines Stechbeckens 14 in allen erforderlichen Ansichten dargestellt. Diese aus der Faßreinigung bekannte Sprühvorrichtung 13 weist eine Birnenform auf und ist vorliegend mit insgesamt vier Sprühschlitzen 33, 34, 35, 36 versehen. Davon verlaufen die beiden Sprühschlitze 33, 34 exzentrisch zur Drehachse 28 des Sprühkopfes 13, wohingegen die beiden weiteren Sprühschlitze 35, 36 auf einer Ebene mit der Drehachse 28 liegen. Der birnenförmige Sprühkopf 13 ist auf einem zylinderförmigen Sockel 37 frei drehbar. Aufgrund seiner geringen Masse wird bei Druckbeaufschlagung mit Reinigungswasser dieser Sprühkopf 13 aufgrund der von den Sprühschlitzen 33, 34 erzeugten Rückstoßimpulse mit hoher Drehzahl gedreht. Diese Drehzahl ist wesentlich höher als die bei den bisher bekannten Geräten zur Reinigung und Desinfektion aus zwei Sprüharmen bestehenden Sprühvorrichtungen. Da außerdem der Druckverlust der durch die Sprühschlitze 33 - 36 des austretenden Reinigungswassers erheblich geringer als bei den nach dem Stand der Technik bekannten Sprühvorrichtungen ist, trifft das austretende Reinigungswasser mit hoher Aufprallgeschwindigkeit auf die Innenflächen des Stechbeckens 14, insbesondere auf dessen Boden 14a.

Die etwa senkrecht auf den dreieckförmigen Seitenflächen 24, 25 des Verteilergehäuses 16 angeordneten weiteren Sprühköpfe 29, 30 sind in den Figuren 14 bis 17 dargestellt. Diese Sprühköpfe 29, 30 sind mit einer Strahl-Leitvorrichtung 38 versehen, die im wesentlichen aus einer Auskehlung eines sich an die Austrittsöffnung 39 anschließenden zylindrischen Fortsatzes 40 gebildet ist. Dadurch wird der aus der Öffnung 39 austretende Wasserstrahl durch die Strahl-Leitvorrichtung 38 in Richtung des Pfeils 41 derart umgelenkt, daß er gezielt die Außenflächen 14b und 14c des Stechbeckens 14 trifft, und zwar beidseitig, weil zwei dieser Sprühköpfe 29, 30 am Verteilergehäuse 16 angeordnet sind. Der weitere Sprühkopf 32 in Nähe der Deckenwand 7 des Gerätegehäuses 2 gewährt außerdem noch eine Besprühung der Außenflächen 14b, 14c des Stechbeckens 14 von oben.

Das Kernstück der Erfindung ist darin zu sehen, daß einerseits in einem kompakten, als Druckkammer ausgebildeten Verteilergehäuse 16 die Sprühdüsen 10 ÷ 12 und die Sprühköpfe 29 und 30 sowie die gleichfalls als Sprühkopf ausgebildete Sprühvorrichtung 13 angeordnet sind und daher gleichmäßig mit dem gleichen Druck beaufschlagt werden. Ein gering verminderter Druck kann lediglich an der Sprühdüse 32 oder einem auch dort angeordneten weiteren Sprühkopf 13 auftreten, weil die Leitung 31 einen geringen, jedoch nicht störend erscheinenden Druckverlust hervorrufen kann.

Da nunmehr gemäß Figur 3 nur noch zwei Anschlußstutzen, nämlich der Anschlußstutzen 18 für Wasser und der Anschlußstutzen 19 für Dampf auf der Rückseite 3a der Rückwand 3 anzuschließen und abzudichten sind, entfällt die bisherige Problematik einer stets gesicherten Abdichtung einer "Rohrspinne" mit zahlreichen Verschraubungen und Quetschverbindungen. Dies führt neben einem erheblich verminderten Bau- und Montageaufwand auch zu einer geringeren Anfälligkeit gegen Leckagen, zu einer weitgehenden Wartungsfreiheit des gesamten Gerätes 1 sowie ebenso zu einer äußerst effektiven und gründlichen Reinigung der im Innenraum 9 des Gerätegehäuses 2 angeordneten Stechbecken 14 und der Urinflaschen auf ihren sämtlichen Innen- und Außenflächen. Dabei können die Sprühdüsen 29 und 30 mit ihren Strahl-Leitvorrichtungen 38 derart einjustiert werden, daß auch andere Behälter als Stechbecken 14, z. B. Töpfe von Stuhlklosetts gründlich auf ihren Außenflächen gereinigt werden.

### Bezugszeichenliste

- Gerät: 1
- Gerätegehäuse: 2
- Rückwand: 3
- Außenseite von Rückwand 3: 3a
- Innenseite von Rückwand 3: 3b
- Seitenwände: 4, 5
- Bodenwand: 6
- Deckenwand: 7
- Frontklappe: 8
- Innenraum von Gerätegehäuse 2: 9
- Sprühdüsen: 10, 11, 12; 32
- Sprühvorrichtung: 13
- Stechbecken: 14
- Bodenfläche von Stechbecken 14: 14a
- Außenflächen von Stechbecken 14: 14b, 14c
- Dampfeinlaß: 15
- Verteilergehäuse/Druckkammer: 16
- Anschlußseite von Verteilergehäuse 16: 17
- Anschlußstutzen für Wasser: 18
- Anschlußstutzen für Dampf: 19
- Rohr für Dampfdurchlaß: 20
- Frontseite von Verteilergehäuse 16: 21
- Rückseite von Verteilergehäuse 16: 22
- Querschnitts-Dreieckseite: 23
- Basisseite von Querschnitts-Dreieckseite 23: 23a
- Dreiecksflächen von Verteilergehäuse 16: 24, 25
- Schenkelseiten von Dreiecksflächen 24, 25: 26, 27
- Drehachse von Sprühvorrichtung 13: 28
- Sprühköpfe an Dreiecksflächen 24, 25: 29, 30
- Leitung aus Verteilergehäuse 16: 31
- Sprühdüse an Leitung 31: 32
- Sprühschlitze an Sprühvorrichtung 13: 33, 34, 35, 36
- zylinderförmiger Sockel von Sprühvorrichtung 13: 37
- Strahl-Leitvorrichtung: 38
- Austrittsöffnung von Strahl-Leitvorrichtung 38: 39
- zylindrischer Fortsatz von Strahl-Leitvorrichtung 38: 40
- Pfeil: 41

## Patentansprüche

1. Gerät zur Reinigung und Desinfektion mit einem Gerätegehäuse (2), bestehend aus einer Rückwand (3), zwei Seitenwänden (4, 5) einer Boden (6) und einer Deckenwand (7) sowie mit einer verschließbaren Frontklappe (8), mit im Innenraum angeordneten Sprühdüsen zum Reinigen von Urinflaschen und mit einer sich nach dem Prinzip eines Segnerschen Wasserrades durch Rückstoßimpulse drehenden Sprühvorrichtung zur Reinigung eines Stechbeckens (14) sowie mit einem Dampfeinlaß zur Desinfektion, **dadurch gekennzeichnet, daß** im Innenraum (9) des Gerätegehäuses (2) ein kompaktes Verteilergehäuse (16) angeordnet ist, welches als Druckkammer (16) ausgebildet ist und mit mehreren Sprühdüsen (10 ÷ 12; 32) und mit mindestens einer Sprühvorrichtung (13) versehen ist sowie an seiner Rückseite (22) zwei Anschlußstutzen (18, 19) für Wasser und Dampf aufweist, die abgedichtet durch die Rückwand (3) des Gerätegehäuses (2) nach außen hindurchgeführt und auf der Außenseite (3a) dieser Rückwand (3) mit nur einer Wasser- und einer Dampfzufuhr strömungsleitend verbunden sind.

2. Gerät nach Anspruch 1, **dadurch gekennzeichnet, daß** der Anschlußstutzen (19) für die Dampfzufuhr druckdicht mit einem Rohr (20) verbunden ist, welches dampf- und wasserdicht durch die Druckkammer (16) hindurchgeführt ist und an einer abgedichteten Öffnung (15) an deren Frontseite (21) endet.

3. Gerät nach Anspruch 2, **dadurch gekennzeichnet, daß** das Rohr (20) vom Anschlußstutzen (19) zur Frontseite (21) der Druckkammer (16) in Richtung auf die Bodenwand (6) des Gerätegehäuses (2) geneigt ist.

4. Gerät nach einem der Ansprüche 1 bis 3, **dadurch gekennzeichnet , daß** der Anschlußstutzen (18) für die Wasserzufuhr abgedichtet in den Innenraum der Druckkammer (16) geleitet ist, in welcher bei Beaufschlagung mit Reinigungswasser ein gleichmäßiger Druck über deren gesamten Innenraum aufgebaut ist.

5. Gerät nach einem der Ansprüche 1 bis 4, **dadurch gekennzeichnet , daß** das Verteilergehäuse (16) im Querschnitt eine prismatische Dreieckform aufweist, dessen Basisseite (22) mit den Anschlußstutzen (18, 19) für Wasser und Dampf versehen ist sowie auf der Innenseite (3b) der Gehäuserückwand (3) befestigt ist.

6. Gerät nach einem der Ansprüche 1 bis 5, **dadurch gekennzeichnet, daß** die den Urinflaschen und dem Stechbecken (14) zugewandte eine Querschnitts-Dreieckseite (23) des Verteilergehäuses (16) eine Trapezform aufweist, dessen längere Basisseite (23a) der Rückwand (3) des Gerätegehäuses (2) zugekehrt ist.

7. Gerät nach einem der Ansprüche 5 bis 6, **dadurch gekennzeichnet, daß** die Frontseite (21) des Verteilergehäuses (16) rechteckförmig ausgebildet ist.

8. Gerät nach einem der Ansprüche 5 bis 7, **dadurch gekennzeichnet, daß** die beiden Seitenflächen (24, 25) des Verteilergehäuses (16) dreieckförmig ausgebildet sind, und die kürzeren Schenkelseiten (26, 27) zugleich die Schmalseiten (26, 27) der Frontseite (21) bilden.

9. Gerät nach einem der Ansprüche 6 bis 8, **dadurch gekennzeichnet, daß** die Sprühvorrichtung (13) auf der trapezeodalen Oberseite (23) des Verteilergehäuses (16) als Sprühkopf (13) ausgebildet ist und eine Birnenform aufweist, die mit mindestens vier Sprühschlitzen (33 ÷ 36) versehen ist, von denen zwei (33, 34) parallel, jedoch exzentrisch zur Drehachse (28) des Sprühkopfes (13) verlaufen.

10. Gerät nach Anspruch 9, **dadurch gekennzeichnet, daß** die zwei weiteren Sprühschlitze (35, 36) diametral am Sprühkopf (13) angeordnet sind und mit der Drehachse (28) in einer Ebene liegen.

11. Gerät nach einem der Ansprüche 6 bis 10, **dadurch gekennzeichnet, daß** die trapezförmige Oberseite (23) des Verteilergehäuses (16) eine solche Neigung in Richtung auf die an der Frontklappe (8) gehalterten Stechbecken (14) und Urinflaschen aufweist, daß der Sprühkopf (13) senkrecht zur Bodenfläche (14a), des Stechbeckens (14) und die Sprühdüsen (10 ÷ 12) senkrecht zur Bodenfläche der jeweiligen Urinflasche ausgerichtet sind.

12. Gerät nach Anspruch 11, **dadurch gekennzeichnet, daß** aus dem Verteilergehäuse (16) mindestens eine Leitung (31) mit mindestens einer Sprühdüse (32) und/oder einem Sprühkopf (13) zur Deckenwand (7) des Gerätegehäuses (2) geführt ist.

13. Gerät nach einem der Ansprüche 8 bis 12, **dadurch gekennzeichnet, daß** etwa senkrecht auf den dreieckförmigen Seitenflächen (24, 25) des prismatischen Verteilergehäuses (16) zwei Sprühköpfe (29, 30) mit einer Strahl-Leitvorrichtung (38) für den austretenden Wasserstrahl angeordnet sind.

14. Gerät nach Anspruch 13, **dadurch gekennzeichnet, daß** der Wasserstrahl dieser Sprühköpfe (29, 30) auf die Außenseiten (14b. 14c) des Stechbeckens (14) gerichtet ist.

15. Gerät nach einem der Ansprüche 1 bis 14, **dadurch gekennzeichnet, daß** das Verteilergehäuse (16) aus Edelstahl hergestellt ist.

16. Gerät nach einem der Ansprüche 1 bis 15, **dadurch gekennzeichnet, daß** die in die entsprechenden Öffnungen des Verteilergehäuses (16) abgedichtet eingeschraubten Sprühdüsen (10 ÷ 12; 32) sowie die Sprühköpfe (13; 29, 30) entweder aus Edelstahl oder aus einer verchromten Messinglegierung bestehen.

## Claims

1. A device for cleaning and disinfection comprising a device housing (2) consisting of a rear wall (3), two side walls (4, 5), a bottom (6) and a top wall (7) as well as a closable front flap (8) with spray nozzles for cleaning urine bottles located in the interior and comprising a spray device for cleaning a bedpan (14) which rotates as a result of recoil pulses according to the principle of a Segner waterwheel, and comprising a steam inlet for disinfection, **characterised in that** located in the interior (9) of the device housing (2) is a compact distributor housing (16) which is configured as a pressure chamber (16) and is provided with a plurality of spray nozzles (10-12; 32) and with at least one spray device (13) and which has two pipe connections (18, 19) for water and steam on its back (22) which are guided outside through the rear wall (3) of the device housing (2) in a sealed manner and on the outer side (3a) of this rear wall (3) are only connected to a water and a steam supply in a flow-conducting manner.

2. The device according to claim 1, **characterised in that** the pipe connection (19) for the steam supply is connected in a pressure-tight manner to a pipe (20) which is guided in a steam- and water-tight manner through the pressure chamber (16) and ends at a sealed opening (15) on the front side (21) thereof.

3. The device according to claim 2, **characterised in that** the pipe (20) is inclined from the pipe connection (19) to the front side (21) of the pressure chamber (16) in the direction of the bottom wall (6) of the device housing (2).

4. The device according to any one of claims 1 to 3, **characterised in that** the pipe connection (18) for the water supply is guided in a sealed manner into the interior of the pressure chamber (16) in which a uniform pressure is built up over the entire interior when this is acted upon with cleaning water.

5. The device according to any one of claims 1 to 4, **characterised in that** the distributor housing (16) has a prismatic triangular shape in cross-section, the base side (22) whereof is provided with the pipe connections (18, 19) for water and steam and is affixed to the inner side (3b) of the housing rear wall (3).

6. The device according to any one of claims 1 to 5, **characterised in that** the one triangular cross-section side (23) of the distributor housing (16) facing the urine bottle and the bed pan (14) has a trapezoidal shape whose longer base side (23a) is facing the rear wall (3) of the device housing (2).

7. The device according to any one of claims 5 to 6, **characterised in that** the front side (21) of the distributor housing (16) is configured as rectangular.

8. The device according to any one of claims 5 to 7, **characterised in that** the two side surfaces (24, 25) of the distributor housing (16) are configured as triangular and the shorter sides (26; 27) at the same time form the narrow sides (26, 27) of the front side (21).

9. The device according to any one of claims 6 to 8, **characterised in that** the spray device (13) is formed as a spray head (13) on the trapezoidal upper side (23) of the distributor housing (16) and has a pear shape which is provided with at least four spray slots (33 - 36) of which two run eccentrically to the axis of rotation (28) of the spray head (13).

10. The device according to claim 9, **characterised in that** the two further spray slots (35, 36) are arranged diametrically on the spray head (13) and lie in one plane with the axis of rotation (28).

11. The device according to any one of claims 6 to 10, **characterised in that** the trapezoidal upper side (23) of the distributor housing (16) has such a slope in the direction of the bedpan (14) and urine bottles held at the front flap (8) that the spray head (13) is aligned perpendicular to the bottom surface (14a) of the bedpan (14) and the spray nozzles (10-12) are aligned perpendicular to the bottom surface of the respective urine bottle.

12. The device according to claim 11, **characterised in that** at least one line (31) with at least one spray nozzle (32) and/or one spray head (13) is guided from the distributor housing (16) towards the top wall (7) of the device housing (2).

13. The device according to any one of claims 8 to 12, **characterised in that** two spray heads (29, 30) with a jet guiding device (38) for the emerging water jet are arranged approximately perpendicularly on the triangular side surfaces (24, 25) of the prismatic distributor housing (16).

14. The device according to claim 13, **characterised in that** the water jet from these spray heads (29, 30) is directed onto the outer sides (14b, 14c) of the bedpan (14).

15. The device according to any one of claims 1 to 14, **characterised in that** the distributor housing (16) is made of stainless steel.

16. The device according to any one of claims 1 to 15, **characterised in that** the spray nozzles (10-12; 32) screwed into the corresponding openings of the distributor housing (16) in a sealed manner as well as the spray heads (13; 29, 30) are either made of stainless steel or of a chrome-plated brass alloy.

## Revendications

1. Dispositif de nettoyage et de désinfection, avec un boîtier d'appareil (2) consistant en une paroi arrière (3), en deux parois latérales (4, 5), en un fond inférieur (6) et en un fond supérieur (7), ainsi qu'avec un volet frontal (8) fermable, avec des buses de pulvérisation disposées à l'intérieur, pour le nettoyage de pistolets et avec un dispositif de pulvérisation en rotation par impulsions de réaction, selon le principe d'un tourniquet hydraulique de Segner, pour le nettoyage d'un bassin (14), ainsi qu'avec une entrée de vapeur pour la désinfection, **caractérisé en ce qu'**à l'intérieur (9) du boîtier de l'appareil (2) est disposé un boîtier distributeur compact (16) conçu sous la forme d'une chambre de compression (16) et muni de plusieurs buses de pulvérisation (10 à 12 ; 32) et d'au moins un dispositif de pulvérisation (13) et comportant sur sa face arrière (22) deux raccords (18, 19) pour l'eau et la vapeur, qui sont tirés vers l'extérieur en étant rendus étanches, à travers la paroi arrière (3) du boîtier d'appareil (2) et qui sur la face extérieure (3a) de ladite paroi arrière (3) sont reliés de façon conductrice du flux avec seulement une alimentation en eau et une alimentation en vapeur.

2. Appareil selon la revendication 1,
**caractérisé en ce que** le raccord (19) pour l'alimentation de vapeur est relié de façon étanche à la pression avec un tube (20) qui est guidé de façon étanche à la vapeur et à l'eau à travers la chambre de compression (16) et qui se termine sur une ouverture (15) rendue étanche sur sa face frontale (21).

3. Appareil selon la revendication 2, **caractérisé en ce que** le tube (20) du raccord (19) est incliné vers la face frontale (21) de la chambre de compression (16), en direction du fond inférieur (6) du boîtier d'appareil (2)

4. Appareil selon l'une quelconque des revendications 1 à 3, **caractérisé en ce que** le raccord (18) pour l'alimentation d'eau est conduit en étant isolé vers l'intérieure de la chambre de compression (16), dans laquelle, lorsqu'elle est soumise à de l'eau de nettoyage se créé une pression régulière sur tout son intérieur.

5. Appareil selon l'une quelconque des revendications 1 à 4, **caractérisé en ce que** le boîtier distributeur (16) présente en section transversale une forme triangulaire prismatique, donc le côté de la base (22) est muni des raccords (18, 19) pour l'eau et la vapeur et est fixé sur la face intérieure (3b) de la paroi arrière du boîtier (3).

6. Appareil selon l'une quelconque des revendications 1 à 5, **caractérisé en ce que** l'un des côtés du triangle de la section transversale (23) faisant face aux pistolets et au bassin (14) du boîtier distributeur (16) a une forme trapézoïdale, dont le plus long côté de la base (23a) fait face à la paroi arrière (3) du boîtier d'appareil (2).

7. Appareil selon l'une quelconque des revendications 5 à 6, **caractérisé en ce que** la face frontale (21) du boîtier distributeur (16) est conçue en forme de rectangle.

8. Appareil selon l'une quelconque des revendications 5 à 7, **caractérisé en ce que** les deux surfaces latérales (24, 25) du boîtier distributeur (16) sont conçues en forme de triangle et **en ce que** les côtés les plus courts des branches (26, 27) forment simultanément les côtés étroits 26, 27) de la face frontale (21).

9. Appareil selon l'une quelconque des revendications 6 à 8, **caractérisé en ce que** sur la face supérieure trapézoïdale (23) du boîtier distributeur (16), le dispositif de pulvérisation (13) est conçu en tant que tête de pulvérisation (13) et a la forme d'une poire, qui est munie d'au moins quatre fentes de pulvérisation (33 à 36) dont deux (33, 34) s'étendent à la parallèle, mais de façon excentrique par rapport à l'axe de rotation (28) de la tête de vaporisation (13).

10. Appareil selon la revendication 9, **caractérisé en ce que** les deux autres fentes de vaporisation (35, 36) sont disposées de façon diamétrale sur la tête de vaporisation (13) et se situent dans un plan avec l'axe de rotation (28).

11. Appareil selon l'une quelconque des revendications 6 à 10, **caractérisé en ce que** la face supérieure trapézoïdale (23) du boîtier distributeur 16) présente une inclinaison telle, en direction des bassins (14) et pistolets fixés sur le volet frontal (8) que la tête de vaporisation (13) est orientée à la perpendiculaire de la surface du fond (14a) du bassin (14) et que les buses de vaporisation (10 à 12) sont orientées à la perpendiculaire de la surface du fond du pistolet concerné.

12. Appareil selon la revendication 11, **caractérisé en ce que** à partir du boîtier distributeur (16), au moins un conduit (31) avec au moins une buse de vaporisation (32) et/ou une tête de vaporisation (13) est tiré vers le fond supérieur (7) du boîtier d'appareil (2).

13. Appareil selon l'une quelconque des revendications 8 à 12, **caractérisé en ce que**, environ à la perpendiculaire sur les surfaces latérales triangulaires (24, 25) du boîtier distributeur prismatique (16) sont disposées deux têtes de vaporisation (29, 30) avec un dispositif de guidage du jet (38) pour le jet d'eau sortant.

14. Appareil selon la revendication 13, **caractérisé en ce que** le jet d'eau desdites têtes de vaporisation (29, 30) est orienté vers les faces extérieures (14b, 14c) du bassin (14).

15. Appareil selon l'une quelconques des revendications 1 à 14, **caractérisé en ce que** le boîtier distributeur (16) est fabriqué en acier surfin.

16. Appareil selon l'une quelconque des revendications 1 à 15, **caractérisé en ce que** les buses de vaporisation (10 à 12 ; 32) vissées de façon étanche dans les ouvertures correspondantes du boîtier distributeur (16), ainsi que les têtes de vaporisation (13 ; 29, 30) sont soit en acier surfin ou en un alliage de laiton chromé.
